(19) Europäisches Patentamt
European Patent Office
Office européen des brevets

(11) Numéro de publication: **0 197 829**
**A1**

(12) **DEMANDE DE BREVET EUROPEEN**

(21) Numéro de dépôt: 86400604.4

(22) Date de dépôt: 21.03.86

(51) Int. Cl.⁴: C 07 D 471/14
A 61 K 31/435
//(C07D471/14, 221:00, 221:00, 209:00)

(30) Priorité: 22.03.85 FR 8504872

(43) Date de publication de la demande:
15.10.86 Bulletin 86/42

(84) Etats contractants désignés:
AT BE CH DE FR GB IT LI LU NL SE

(71) Demandeur: SANOFI
40, Avenue George V
F-75008 Paris(FR)

(71) Demandeur: CENTRE NATIONAL DE LA RECHERCHE SCIENTIFIQUE (CNRS)
15, Quai Anatole France
F-75700 Paris(FR)

(72) Inventeur: Bisagni, Emile
16 rue Bossuet
F-91400 Orsay(FR)

(72) Inventeur: Chi Hung, Nguyen
7 allée des Amonts
F-91940 Les Ulis(FR)

(72) Inventeur: Pepin, Odile
23 rue St Eloi
F-31400 Toulouse(FR)

(74) Mandataire: Polus, Camille et al,
c/o Cabinet Lavoix 2, Place d'Estienne d'Orves
F-75441 Paris Cedex 09(FR)

(54) **Nouveaux dérivés de la 5-H pyrido (3',4':4,5) pyrolo (3,2-c) pyridine, leur procédé de préparation et leur activité antitumorale.**

(57) La présente invention est relative à de nouveaux dérivés de la 5-H pyrido[3', 4':4, 5]pyrrolo[3, 2-c]pyridine de formule:

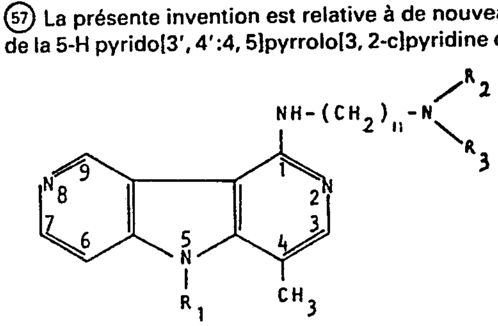

dans laquelle n représente un nombre entire de 2 à 4, $R_1$ représente l'hydrogène ou un groupe alcoyle inférieur, $R_2$ et $R_3$ représentent l'hydrogène, un groupe alcoyle inférieur ou hydroxyalcoyle ainsi que les formes tautomères et les sels d'addition avec les acides minéraux ou organiques pharmaceutiquement acceptables.

L'invention a également pour objet leur pocédé de préparation et leur application en thérapeutique en tant qu'antitumoraux.

EP 0 197 829 A1

Croydon Printing Company Ltd

Nouveaux dérivés de la 5-H pyrido[3',4':4,5]pyrrolo[3,2-c]-pyridine, leur procédé de préparation et leur activité antitumorale.

L'invention est relative à de nouveaux dérivés de la 5-H pyrido[3',4':4,5]pyrrolo[3,2-c]pyridine, à un procédé pour les préparer et à leurs applications en thérapeutique.

Ces composés qui présentent des propriétés antitumorales intéressantes, répondent à la formule suivante :

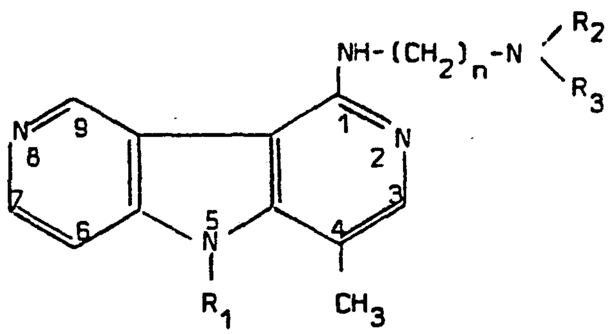

(I)

dans laquelle n représente un nombre entier de 2 à 4, $R_1$ représente l'hydrogène ou un groupe alcoyle inférieur, $R_2$ et $R_3$ sont chacun indépendamment l'un de l'autre, l'hydrogène, un groupe alcoyle inférieur ou hydroxyalcoyle. L'invention concerne aussi les formes tautomères de formule (I) lorsqu'elles existent ainsi que les sels d'addition avec les acides minéraux ou organiques pharmaceutiquement acceptables.

Par radical alcoyle inférieur, on entend une chaîne hydrocarbonée saturée en $C_1$ - $C_4$ linéaire ou ramifiée.

L'invention a également pour objet un procédé de préparation des composés de formule (I) ci-dessus caractérisé en ce que :

a) par réaction de l'hydrazine hydratée sur l'hydroxy-4 méthyl-5 1-H pyridone-2, on prépare l'hydrazino-4 pyridone-2 de formule (II) :

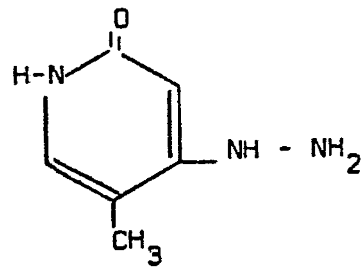

(II)

b) on fait réagir l'hydrazine de formule (II) sur la N-acétyl pipéridone-4 pour obtenir l'hydrazone correspondante de formule (III) :

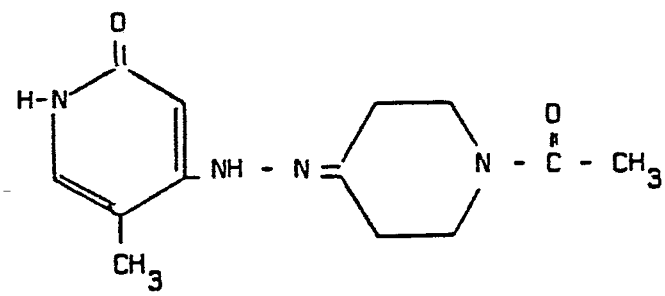

(III)

c) par la réaction de Fisher, on cyclise l'hydrazone de formule (III) pour former la tétrahydro-6,7,8,9 acétyl-8 méthyl-4 2-H, 5-H pyrido [3',4':4,5]pyrrolo[3,2-c]pyridone-1 de formule (IV) :

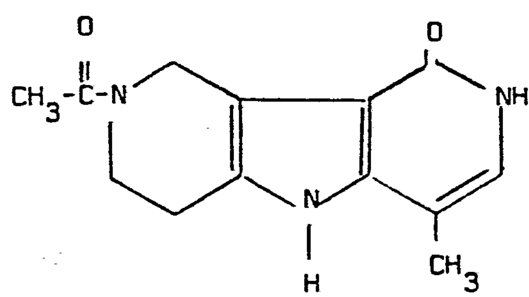

(IV)

d) on aromatise le composé de formule (IV) pour obtenir le méthyl-4 2-H, 5-H pyrido[3',4':4,5]pyrrolo[3,2-c]pyridone-1 de fromule (V) :

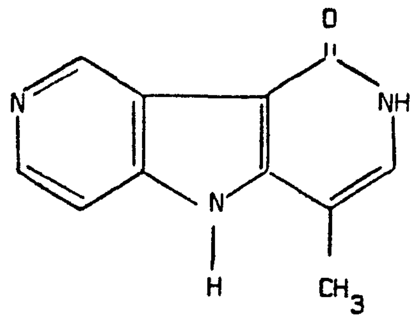

(V)

e) on soumet le composé de formule (V) à une chloration et ensuite, si nécessaire, à une réaction d'alcoylation pour obtenir un dérivé chloro-1 méthyl-4 5-H pyrido[3',4':4,5]pyrrolo[3,2-c]pyridine de formule (VI) :

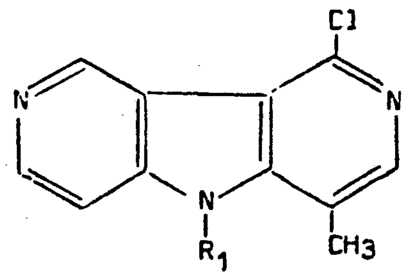

(VI)

dans lequel $R_1$ est tel que défini ci-dessus ; et

f) on condense sur le composé de formule (VI) l'amine de formule $NH_2-(CH_2)_n-N{<}^{R_2}_{R_3}$ (n, $R_2$ et $R_3$ tels que définis ci-dessus) pour obtenir le composé de formule (I).

Le produit de départ, l'hydroxy-4 méthyl-5 1-H pyridone-2 est un composé connu dont la synthèse a été décrite (E. BISAGNI, N.C. HUNG, Synthesis, 1984, p. 765).

La formation du composé de formule (II) s'effectue à une température comprise entre 100°C et 150°C au reflux dans un solvant inerte tel que l'éther mono éthylique de l'éthylèneglycol.

La formation du composé de formule (III) est effectuée par réaction rapide au reflux d'éthanol absolu.

La cyclisation du composé de formule (III) est obtenue par chauffage à des températures comprises entre 200°C et 280°C au reflux d'un solvant inerte tel que le

diphényl éther.

L'aromatisation du composé de formule (IV) pour obtenir le composé de formule (V) s'effectue dans le même solvant que celui mis en oeuvre dans l'étape précédente, à haute température, en présence d'un catalyseur tel que le charbon palladié. Il n'est donc pas utile, dans cette opération, d'isoler le composé de formule (IV) intermédiaire, l'aromatisation s'effectuant simplement par l'introduction dans le milieu réactionnel du catalyseur, l'ensemble des deux réactions -cyclisation et aromatisation étant de préférence réalisé en atmosphère inerte.

La chloration du composé de formule (V) est obtenue par l'action du dichlorure de l'acide phénylphosphonique à une température comprise entre 140°C et 200°C.

L'alcoylation éventuelle du dérivé de formule (VI) s'effectue tout d'abord en préparant un dérivé métallé (sodé ou lithié) par action soit de l'hydrure de sodium soit d'un organolithien tel que le butyllithium, dans un solvant polaire aprotique approprié, à une température comprise entre -70°C et 10°C, puis en faisant réagir un halogénure d'alcoyle $R_1$-hal ($R_1$ tel que défini ci-dessus et hal représentant le chlore, le brome ou l'iode) à une température comprise entre -50°C et 30°C.

L'amination du composé de formule (VI) qui conduit au composé de formule (I), est obtenue par action d'une amine de formule :

$$NH_2 - (CH_2)_n - N\begin{matrix} R_2 \\ R_3 \end{matrix}$$

dans laquelle n, $R_2$ et $R_3$ ont les significations précitées, à une température comprise entre 120°C et 200°C, correspondant en général, à la température de reflux de cette amine.

Les exemples non limitatifs suivants sont donnés à titre d'illustration de l'invention.

Exemple 1.

Préparation de la chloro-1 méthyl-4 5-H pyrido[3',4':4,5]-pyrrolo[3,2-c]pyridine (composé de formule (VI) dans lequel $R_1$ = H.

A) Hydrazino-4 méthyl-5 1-H pyridone-2 (composé de formule (II)).

Le mélange formé par l'hydroxy-4 méthyl-5-1H pyridone-2 (16 g), l'éther monoéthylique de l'éthylène-glycol (400 ml) et l'hydrate d'hydrazine (140 ml) est chauffé au reflux pendant 4 jours et évaporé à sec sous pression réduite. Le résidu solide est repris dans 500 ml d'éthanol absolu bouillant, filtré et le filtrat est concentré de moitié.

Après une nuit à la température ambiante, le solide cristallisé est essoré et séché. On obtient des cristaux incolores (13 g) correspondant à l'hydrate du composé recherché, F = 135-155°C.

Calc. pour $C_6H_9N_3O,H_2O$ = C, 45,85 ; H, 7,05 ; N, 26,74.
Trouvé : C, 45,29 ; H, 6,97 ; N, 26,03.

Les eaux mères concentrées à 100 ml et abandonnées une nuit à la température ambiante fournissent une nouvelle quantité du composé (2,4 g). Le rendement total s'élève donc à 15,4 g, soit 76 p. 100.

RMN $H_1$ [$(CD_3)_2$ SO] ; δ : 1,84 (d, 3H, $CH_3$, $J_{CH_3-H-6}$ = 1 Hz); 4,06 (m, 2H, $NH_2$) ; 5,57 (s, 1H, H-3) ; 6,81 (d, 1H, H-6) ; 7,04 (s, 1H, -N<u>H</u> - $NH_2$) ; 10,13 (s, 1H, NH-1).

B) Acétyl-1 [(dihydro-1,2 méthyl-5 oxo-2 pyridyl-4) hydrazono]-4 pipéridine (composé de formule (III)).

Le mélange formé par l'hydrazine (II) (15 g, 10,7 mmoles), la N-acétyl-pipéridone-4 (18 g, 15,9 mmoles) dans l'éthanol absolu (400 ml) est chauffé à reflux pendant 1 h 30, laissé 15 h à la température ambiante et refroidi à 0°C pour donner un premier précipité qui est filtré et séché. Les eaux mères, concentrées à 150 ml et laissées

15 h à la température ambiante fournissent une nouvelle quantité de cristaux. Au total, il se forme ainsi 24 g (85 p. 100) de microcristaux incolores correspondant à l'hydrate de l'hydrazone - F > 260°C.

Calc. pour $C_{13}H_{18}N_4O_2$, $H_2O$ : C, 55,70 ; H, 7,19 ; N, 19,99.
Trouvé : C, 56,04 ; H, 7,15 ; N, 19,62.

C) Tétrahydro-6,7,8,9 méthyl-4 acétyl-8-2H,5H-pyrido[3',4':4,5]pyrrolo[3,2-c]pyridone-1 (composé de formule (IV)).

Le composé précédemment obtenu (2 g) est ajouté au diphényl éther (70 ml) et le mélange, placé sous atmosphère d'azote, est chauffé à reflux. Le solide se dissout peu à peu, le mélange réactionnel passe par une phase presque homogène puis un précipité réapparaît progressivement. Le composé de départ est totalement transformé en 25 min. Le xylène (140 ml) est ajouté au mélange refroidi et le précipité formé est repris dans l'éthanol bouillant et filtré. Après addition de xylène (250 ml) le précipité obtenu est essoré. La concentration des eaux mères, jusqu'à élimination de tout l'éthanol, fournit, après refroidissement, une nouvelle quantité du produit attendu. Au total, il se forme ainsi 1,66 g (88,7 p. 100) de microcristaux incolores, partiellement hydratés.

Rendement : 88,7 p. 100 ; F > 260°C.

Calc. pour $C_{13}H_{15}N_3O_2$, 0,25 $H_2O$ : C 62,52; H 6,21; N 16,83.
Trouvé : C 62,78; H 6,27; N 16,52.

RMN $H_1$ [$(CD_3)_2$ SO] ; δ 2,11 et 2,14 (2s, 2 x 3H, $CH_3$-4 et $CH_3$-CO-) ; 2,53-2,9 (m, 2H, $CH_2$-6) ; 3,78 (q, 2H, $CH_2$-7) ; 4,72 (s, 2H, $CH_2$-9) ; 6,72 (s, 1H, H-3) ; 10,98 (s, 1H, NH-2) ; 11,22 (s, 1H, NH-5).

D) Méthyl-4-2H, 5H-pyrido[3',4':4,5]pyrrolo[3,2-c]pyridone-1 (composé de formule (V)).

<u>Méthode A</u>. Le composé de formule (IV) précédemment obtenu (120 mg) est chauffé pendant 2 h 30 dans le diphényléther (13 ml) à reflux, en présence de 100 mg de charbon palladié

à 10 p. 100. On ajoute encore 100 mg de charbon palladié, chauffe de nouveau à reflux pendant 1 h 30, laisse refroidir, ajoute 50 ml d'éthanol bouillant et filtre. Le résidu de l'évaporation du filtrat est repris dans l'éther de pétrole et le solide obtenu est recristallisé dans le minimum d'éthanol pour donner 60 mg (56,4 p. 100) de microcristaux incolores correspondant à l'hydrate du composé recherché.

Méthode B. Le mélange formé par l'hydrazone (III) (23,2 g) dans le diphényléther (1,4 l) est maintenu sous agitation sous atmosphère d'argon et chauffé à reflux pendant 30 min. On ajoute 4 g de charbon palladié à 30 p. 100 en suspension dans 100 ml de diphényléther et chauffe l'ensemble à reflux, sous agitation et sous argon, pendant 2 h. Après addition d'1 g de charbon palladié, le chauffage est encore poursuivi pendant 1 h et le mélange réactionnel est refroidi. Après addition de 2 l d'éthanol, l'ensemble est chauffé à reflux et filtré.

a) L'éthanol est évaporé sous pression réduite et le toluène (1,4 l) est ajouté au diphényl éther résiduel. Le précipité formé est essoré, repris dans 150 ml d'éthanol bouillant et refroidi pour donner 5,6 g du composé recherché.

b) Le mélange solide filtré est repris dans 450 ml de diméthylformamide, filtré à l'ébullition, et le filtrat refroidi fournit 5,4 g du produit.

c) Une nouvelle quantité (2 g) est obtenue après traitement de l'ensemble des eaux mères au charbon animal, filtration et concentration à 150 ml.

Par rapport au produit de départ (l'hydrazone), le rendement global calculé en produit pur, s'élève donc à 73,7 p. 100.

Calc. pour $C_{11}H_9O$, $H_2O$ : C, 60,82 ; H, 5,10 ; N, 19,35.
Trouvé : C, 60,81 ; H, 5,11 ; N, 19,43.

RMN $H_1$ [$(CD_3)_2$ SO] ; δ 2,29 (d, 3H, $CH_3$-4) ; 7,21 (d, 1H, H-3, $J_{H_3-CH_3-4}$ = 1 Hz) ; 7,49 (q, 1H, $J_{6-7}$ = 5,8 Hz, $J_{6-9}$ = 1 Hz) ; 8,38 (d, 1H, H-7) ; 9,26 (d, 1H, H-9) ; 11,10 (s, 1H, NH-5) ; 12 (s, 1H, NH-2).

E) Chloro-1 méthyl-4-5H-pyrido[3',4':4,5]pyrrolo[3,2-c]-pyridine (composé de formule (VI)).

Le mélange constitué par 500 mg du composé obtenu dans l'étape précédente (2,5 mmoles) et 25 ml de dichlorure de l'acide phényl phosphonique est chauffé au bain d'huile à 170°C, sous agitation pendant 68 h et l'excès de dichlorure est évaporé à 130°C sous 2 mm. L'eau (120 ml) est ajoutée au résidu et le mélange chauffé à l'ébullition puis filtré ; l'insoluble est lavé avec 40 ml d'eau bouillante et le filtrat alcalinisé, à froid, par addition d'ammoniaque. Le précipité formé est essoré, séché et recristallisé dans le xylène pour fournir 450 mg (82,3 p. 100) de microcristaux incolores, F = > 270°C.

Calc. pour $C_{11}H_8ClN_3$ : C 60,70; H 3,70; N 19,30; Cl 16,29.
Trouvé : C 60,50; H 3,62; N 19,33; Cl 16,11.

RMN $H_1$ [$(CD_3)_2$ SO] ; δ : 2,57 (s, 3H, $CH_3$) ; 7,65 (q, 1H, H-6, $J_{6-7}$ = 6 Hz, $J_{6-9}$ = 1 Hz) ; 8,2 (s, 1H, H-3) ; 8,63 (d, 1H, H-7) ; 9,54 (d, 1H, H-9) ; 11,86 (s, 1H, NH).

Exemple 2.

Préparation de la chloro-1 diméthyl-4,5,5-H pyrido[3',4':4,5]pyrrolo[3,2-c]pyridine (composé de formule (VI) dans lequel $R_1$ = $CH_3$.

Méthode A.

Le composé chloré de formule (VI) ($R_1$ = H) (5 g) dissous dans un mélange de tétrahydrofuranne sec (THF, 400 ml) et d'hexaméthylphosphorotriamide sec (HMPT, 40 ml) est placé dans un tricol de 1 l maintenu sous atmosphère d'azote et muni d'un thermomètre ainsi que d'un agitateur magnétique. Après avoir refroidi à -65°C, le t-butyllithium

(20,8 ml de la solution commerciale 2,2 M, 2 équivalents) est ajouté lentement et la solution devenue jaune est agitée à -65°C pendant 15 min. L'iodure de méthyle (1,66 ml, 20 p. 100 d'excès) en solution dans 30 ml de THF est ajouté à -65°C et le mélange est agité à cette température pendant 5 h, puis laissé revenir à la température ambiante pendant 15 h.

On ajoute alors 5 ml d'éthanol et le mélange est évaporé à sec en terminant au bain d'huile à 140°C sous 2 mm Hg de pression pour éliminer le HMPT. Le résidu très visqueux est repris dans 100 ml d'une solution d'hydroxyde de sodium 7,5 N et extrait au chlorure de méthylène. Après évaporation, le résidu est chromatographié sur colonne de silice (35 x 2,2 cm) en commençant l'élution avec le mélange chlorure de méthylène-éthanol 95/5 v/v qui élimine des impuretés colorées concentrées dans les premiers tubes. L'élution est continuée avec le même mélange 9/1, jusqu'à disparition de la tâche correspondant au composé recherché. Toutes les fractions contenant ce dernier sont évaporées et le solide résiduel est recristallisé dans le toluène (100 ml après filtration et concentration) pour donner 2,45 g (46 p. 100) du composé recherché. F = 229-231°C.

Calc. pour $C_{12}H_{10}ClN_3$ : C 62,21; H 4,35; N 18,14; Cl 15,30.
Trouvé : C 62,22; H 4,16; N 18,11; Cl 15,09.

RMN $H_1$ ($CDCl_3$) ; δ : 2,8 (s, 3H, $CH_3$-4) ; 4,13 (s, 3H, N-$CH_3$) ; 7,34 (q, 1H, H-6, $J_{6-7}$ = 6 Hz, $J_{6-9}$ = 1 Hz) ; 8,1 (s, 1H, H-3) ; 8,68 (d, 1H, H-7) ; 9,71 (d, 1H, H-9).

Méthode B.

La réaction est conduite suivant la méthode décrite en A, mais en remplaçant le t-butyllithium par la même proportion molaire de n-butyllithium et en opérant à la température de -15°C.

Rendement : 46 p. 100, F = 229-231°C.

Méthode C.

Le composé chloré de formule (VI) ($R_1$ = H) (653 mg) est dissous dans 60 ml de N,N-diméthylformamide et on ajoute 173 mg d'hydrure de sodium à 50 p. 100 en suspension dans l'huile. Le mélange est ensuite agité à la température ambiante pendant 4 h. puis refroidi à -10°C. A cette température, l'iodure de méthyle (1,2 équivalent) est introduit dans le milieu réactionnel. Après agitation pendant 2 h à -15°C puis pendant 1 h à température ambiante, le solvant est évaporé sous pression réduite. Le produit brut ainsi obtenu est traité comme dans la méthode A pour donner 210 mg du composé.

Rendement : 30,2 p. 100 ; F = 229-231°C.

Exemple 3.

Préparation de la chloro-1 méthyl-4 éthyl-5 5-H pyrido-[3',4':4,5]pyrrolo[3,2-c]pyridine (composé de formule (VI) dans lequel $R_1$ = $C_2H_5$).

Ce composé est préparé selon la même méthode A que dans l'exemple 2 à partir du composé de formule (VI) ($R_1$ = H) et de l'iodure d'éthyle.

Rendement de 37 p. 100. F = 167-168°C.

Calc. pour $C_{13}H_{12}ClN_3$ : C, 63,55 ; H, 4,92 ; N, 17,24.

Trouvé : C, 63,42 ; H, 4,85 ; N, 17,17.

RMN $H_1$ ($CDCl_3$) ; δ : 1,52 (t, 3H, $CH_3-CH_2$) ; 2,79 (s, 3H, $-CH_3$-4) ; 4,62 (q, 2H, $-CH_2-CH_3$) ; 7,45 (d, 1H, H-6, $J_{6-7}$ = 6 Hz) ; 8,18 (s, 1H, H-3) ; 8,71 (d, 1H, H-7) ; 9,75 (s, 1H, H-9).

Exemple 4.

Préparation du [(diéthylamino-3 propyl-1)amino]-1 méthyl-4 5-H pyrido[3',4':4,5]pyrrolo[3,2-c]pyridine, trimaléate - dérivé N° 1.

La chloro-1 méthyl-4 5-H pyrido[3',4':4,5]pyrrolo-[3,2-c]pyridine de formule (VI) ($R_1$ = H) (1 g) est chauffée sous atmosphère d'azote dans la diéthylamino-3 propylamine

(10 g) à 160-170°C pendant 18 h en suivant la disparition du dérivé chloré par chromatographie sur couche mince (silice, éluant : chlorure de méthylène-éthanol 9/1). Puis l'excès d'amine est éliminé sous pression réduite. Le résidu est repris dans 50 ml d'une solution d'hydroxyde de sodium 3N et extrait au chlorure de méthylène. La phase organique est séchée puis concentrée sous pression réduite. Le produit brut est chromatographié sur colonne de silice (chlorure de méthylène-éthanol 95/5). La base ainsi obtenue est dissoute dans une solution acétonique d'acide maléique (au moins 3 équivalents molaires) qui est maintenue à ébullition pendant deux minutes.

Après refroidissement, le trimaléate précipite. Il est filtré et séché.

Rendement 71 p. 100 ; F = 180°C avec décomposition.

Calc. pour $C_{30}H_{37}N_5O_{12}$ : C, 54,62 ; H, 5,65 ; N, 10,62.

Trouvé : C, 54,28 ; H, 5,90 ; N, 10,51.

RMN $H_1$ ($D_2O$) ; δ : 1,38 (t, 2X3H, $\underline{CH_3}$-$CH_2$) ; 2,31 (m, 2H, $CH_2$-β) ; 2,57 (d, 3H, $CH_3$-4) ; 3,18-3,53 (m, 3X2H, $\underline{CH_2}$-$CH_3$ + $CH_2$ - γ) ; 3,85 (t, 2H, $CH_2$ - α) ; 6,27 (s, 6H, $\overline{CH}$ = CH-maléate) ; 7,92 (d, 1H, H-3), $J_3$-$CH_3$-4) = 1 Hz) ; 8,18 (q, 1H, H-6, $J_{6-7}$ = 7 Hz, $J_{6-9}$ = 0,6 H) ; 8,75 (q, 1H, H-7, $J_{7-9}$ = 0,5 Hz) ; 9,77 (d, 1H, H-9).

<u>Exemple 5</u>.

Préparation de l'[[[(hydroxy-2 éthyl-1)amino]-2 éthyl-1]-amino]-1 méthyl-4 5-H pyrido[3',4':4,5]pyrrolo[3,2-c]pyridine - dérivé N° 2.

Ce composé est préparé selon le même mode opératoire que dans l'exemple 4 à partir de la chloro-1 méthyl-4 5-H pyrido[3',4':4,5]pyrrolo[3,2-c]pyridine de formule (VI) ($R_1$ = H) et de l'(hydroxy-2 éthylamino)-2 éthylamine par chauffage pendant 5 h.

Après chromatographie, la base est recristallisée dans l'eau.

Rendement : 80 p. 100 ; F 270°C.

Calc. pour $C_{15}H_{19}N_5O$ ; 2,5 $H_2O$ : C 54,54; H 7,27; N 21,21.

Trouvé : C 54,87; H 6,52; N 20,84.

RMN $H_1$ (($CD_3)_2SO$] : δ : 2,44 (d, 3H, $CH_3$, J $CH_3$-$H_3$ = 1 Hz); 3,11 (t, 2H, NH-$CH_2$-$CH_2$OH) ; 3,29 (t, 2H, -$CH_2$-NH-$CH_2$-$CH_2$OH) ; 3,75 (t, 2H, -$CH_2$OH) ; 3,88 (t, 2H, 1-NH-$CH_2$ -) ; 7,55 (q, 1H, H-6, $J_{6-7}$ = 6 Hz, $J_{6-9}$ = 0,8 Hz) ; 7,88 (d, 1H, H3) ; 8,47 (d, 1H, H-7) ; 9,59 (d, 1H, H-9).

Exemple 6.

Préparation de la [(diméthylamino-3 propyl-1)amino]-1 méthyl-4 5-H pyrido[3',4':4,5]pyrrolo[3,2-c]pyridine, trichlorhydrate - dérivé N° 3.

Ce composé est préparé selon le même mode opératoire que dans l'exemple 4 à partir de la chloro-1 méthyl-4 5-H pyrido[3',4':4,5]pyrrolo[3,2-c]pyridine(de formule (VI)) ($R_1$ = H) et de la diméthylamino-3 propylamine à la température de 180°C pendant 8 heures sous autoclave. Après chromatographie, la base est reprise par une solution éthanolique d'acide chlorhydrique. Le trichlorhydrate précipite ; il est filtré et séché.

Rendement : 70 p. 100 ; se sublime sans fondre à partir de 220°C.

Calc. pour $C_{16}H_{21}N_5$ ; 3 HCl ; 2,5 $H_2O$ :

C, 43,99 ; H, 6,68 ; N, 16 ; Cl, 24,29.

Trouvé : C, 43,70 ; H, 6,52 ; N, 16,24 ; Cl, 24,60.

RMN ($H_1$) ($D_{2O}$) ; δ : 2,39 (m, 2H, $CH_2$-β) ; 2,65 (s, 3H, $CH_3$-4) ; 3,05 (s, 2x3H, N($CH_3)_2$) ; 3,51 (m, 2H, $CH_2$-γ) ; 3,91 (t, 2H, $CH_2$-α) ; 7,99 (s, 1H, H-3) ; 8,29 (d, 1H, H-6, $J_{6-7}$ = 6 Hz) ; 8,81 (d, 1H, H-7) ; 9,92 (s, 1H, H-9).

Dans les mêmes conditions, le trimaléate a été obtenu après reprise de la base par une solution acétonique d'acide maléique (dérivé 3 bis).

Exemple 7.

Préparation de la [(diéthylamino-3 propyl-1)amino]-1 diméthyl-4,5 5-H pyrido[3',4':4,5]pyrrolo[3,2-c]pyridine, trichlorhydrate - dérivé N° 4.

Ce composé a été préparé selon le même mode opératoire que dans l'exemple 4 à partir de la chloro-1 diméthyl-4,5 5-H pyrido[3',4':4,5]pyrrolo[3,2-c]pyridine (composé de formule (VI) $R_1 = CH_3$) et de la diéthylamino-3 propylamine par chauffage pendant 24 h. Après chromatographie, la base est reprise par une solution éthanolique d'acide chlorhydrique. On obtient un précipité de trichlorhydrate qui est filtré et séché.

Rendement 64 p. 100 ; se sublime à partir de 200°C.

Calc. pour $C_{19}H_{27}N_5$ ; 3 HCl ; 3 $H_2O$ :

C, 46,73 ; H, 7,37 ; N, 14,33 ; Cl, 21,80.

Trouvé : C, 46,48 ; H, 7,81 ; N, 13,88 ; Cl, 22,08.

RMN $H_1$ ($D_2O$) ; δ : 1,49 (t, 2x3H, $-CH_2-CH_3$) ; 2,37 (m, 2H, $CH_2$-β) ; 2,9 (d, 3H, $CH_3$-4) ; 3,23-3,56 (m, 3x2H, $-CH_2-CH_3$ + $CH_2$-γ) ; 3,91 (t, 2H, $CH_2$-α) ; 4,46 (s, 3H, N-$CH_3$) ; 7,99 (d, 1H, H-3, $J_{3-CH_3-4}$ = 1 Hz) ; 8,35 (d, 1H, H-6, $J_{6-7}$ = 7 Hz), 8,87 (d, 1H, H-7) ; 9,87 (s, 1H, H-9).

<u>Exemple 8</u>.

Préparation de la [(diéthylamino-3 propyl-1)amino]-1 méthyl-4 éthyl-5 5-H pyrido[3',4':4,5]pyrrolo[3,2-c]pyridine, tétrachlorhydrate - dérivé N° 5.

Ce composé a été préparé selon le même mode opératoire que l'exemple N° 4 à partir de la chloro-1 méthyl-4 éthyl-5 5-H pyrido[3',4':4,5]pyrrolo[3,2-c]pyridine (composé de formule (VI) $R_1$ = éthyl) et la diéthylamino-3 propylamine par chauffage pendant 24 h. Après chromatographie, la base est reprise par une solution éthanolique d'acide chlorhydrique. On recueille un précipité du tétrachlorhydrate par filtration et on le sèche.

Fusion partielle à partir de 170°C.

Calc. pour $C_{20}H_{29}N_5$ ; 4 HCl ; 4 $H_2O$ :

C, 43,09 ; H, 7,36 ; N, 12,57 ; Cl, 25,49.

Trouvé : C, 43,43 ; H, 7,48 ; N, 12,51 ; Cl, 24,70.

RMN $H_1$ ($D_{2}O$) ; δ : 1,45 (t, 2x3H, $N(-CH_2-CH_3)_2$) ; 1,7 (t, 3H, $CH_3-CH_2-N_5$) ; 2,42 (m, 2H, $CH_2$-β) ; 2,92 (s, 3H, $CH_3$-4) ; 3,31-3,6 (m, 3x2H, $N(-CH_2-CH_3)_2$ + $CH_2$-γ) ; 3,95 (t, 2H, $CH_2$-α) ; 5,01 (q, 2H, $CH_3-CH_2-N_5$) ; 8,03 (s, 1H, H-3) ; 8,45 (d, 1H, $H_6$, $J_{6-7}$ = 7 Hz) ; 8,9 (d, 1H, H-7) ; 9,95 (s, 1H, H-9).

Exemple 9.

Préparation de l'[(éthylamino-3 propyl-1) amino]-1 méthyl-4 5-H pyrido[3',4':4,5]pyrrolo[3,2-c]pyridine, trichlorhydrate - dérivé N° 6.

Ce composé a été préparé selon le même mode opératoire que l'exemple N° 4 à partir de chloro-1 méthyl-4 5-H pyrido[3',4':4,5]pyrrolo[3,2-c]pyridine (composé de formule VI dans lequel $R_1$ = -H) et l'éthylamino-3 propylamine par chauffage à 180°C pendant 48 heures.

Après chromatographie, la base est reprise par une solution éthanolique d'acide chlorhydrique. On recueille le précipité sous forme de trichlorhydrate.

Rendement : 65 % ; F > 260°C.

Calc. pour $C_{16}H_{21}N_5$ ; 3 HCl ; 1,2 $H_2O$ :

C, 46,37 ; H, 6,42 ; N, 16,96 ; Cl, 25,66.

Trouvé : C, 46,23 ; H, 6,46 ; N, 17,03 ; Cl, 25,62.

RMN $H_1$ ($D_{2}O$) ; δ : 1,50 (t, 3H, $CH_2-CH_3$) ; 2,40 (m, 2H, $CH_2$-β) ; 2,6 (s, 3H, $CH_3$-4) ; 3,25-3,60 (m, 2x2H, $CH_2-CH_3$ + $CH_2$-γ) ; 4,0 (t, 2H, $CH_2$-α) ; 8,00 (s, 1H, H-3) ; 8,20 (d, 1H, H-6, $J_{6-7}$ = 6,5 Hz) ; 8,90 (d, 1H, H-7) ; 10,05 (s, 1H, H-9).

Exemple 10.

Préparation de [(diéthylamino-2 éthyl-1)amino]-1 méthyl-4 5-H pyrido[3',4':4,5]pyrrolo[3,2-c]pyridine, trichlorhydrate - dérivé N° 7.

Ce composé a été préparé selon le même mode opératoire que l'exemple N° 4 à partir de chloro-1 méthyl-4 5-H pyrido[3',4':4,5]pyrrolo[3,2-c]pyridine (composé de formule VI dans lequel $R_1$ = -H) et la diéthylamino-2 éthylamine par chauffage à 180°C pendant 24 heures.

Après chromatographie, la base est reprise par une solution éthanolique d'acide chlorhydrique. On recueille le précipité sous forme de trichlorhydrate contenant environ 10 % de tétrachlorhydrate.

Rendement : 34 % ; F > 260°C.

Calc. pour $C_{17}H_{23}N_5$ ; 3,1 HCl ; 2,5 $H_2O$ :

C, 45,13 ; H, 6,88 ; N, 15,48 ; Cl, 24,34.

Trouvé : C, 45,28 ; H, 6,45 ; N, 15,29 ; Cl, 24,53.

RMN $H_1$ ($D_2O$) ; δ : 1,40 (t, 2 x 3H, $CH_2$-$CH_3$) ; 2,50 (s, 3H, $CH_2$-4) ; 3,4 (q, 2 x 2H, $\underline{CH_2}$-$CH_3$) ; 3,7 (t, 2H, $CH_2$-β) ; 4,20 (t, 2H, $CH_2$-α) ; 7,90 (s, 1H, H-3) ; 8,20 (d, 1H, $H_6$, J $_{6-7}$ = 7 Hz) ; 8,70 (d, 1H, H-7) ; 9,90 (s, 1H, H-9).

Exemple 11.

Préparation de diméthyl-4,5[(éthylamino-3 propyl-1)amino]-1 5-H pyrido[3',4':4,5]pyrrolo[3,2-c]pyridine, tri et tétramaléate - dérivé N° 8.

Ce composé est préparé selon le même mode opératoire que l'exemple N° 4 à partir de chloro-1 diméthyl-4,5 5-H pyrido[3',4':4,5]pyrrolo[3,2-c]pyridine (composé de formule VI dans lequel $R_1$ = -$CH_3$) et l'éthylamino-3 propylamine au reflux pendant 24 heures. La base obtenue est transformée en tri et tétramaléate (mélange 50/50) par action d'une solution acétonique d'acide maléique en excès.

Rendement : 90 % ; F : 140°C.

Calc. pour $C_{17}H_{23}N_5$ ; 3,5 $C_4H_4O_4$ ; 1,5 $H_2O$ :

C, 50,96 ; H, 5,48 ; N, 9,58.

Trouvé : C, 50,69 ; H, 5,80 ; N, 9,80.

RMN $H_1$ ($D_2O$) ; δ : 1,46 (t, 3H, $CH_2$-$CH_3$) ; 2,38 (m, 2H, $CH_2$-β) ; 2,90 (d, 3H, $CH_3$-4) ; 3,22-3,52 (m, 2 x 2H, $CH_2$-γ + $\underline{CH_2}$-$CH_3$) ; 3,95 (t, 2H, $CH_2$-α) ; 4,48 (s, 3H, $CH_3$-5) ;

6,32 (s, = CH maléate) ; 8,01 (d, 1H, H-3, J H-3-$CH_3$-4 = 1,2 Hz) ; 8,40 (d, 1H, H-6, $J_{6-7}$ = 7 Hz) ; 8,92 (d, 1H, H-7) ; 9,92 (s, 1H, H-9).

Exemple 12.

Préparation de diméthyl-4,5 [(diméthylamino-3 propyl-1)-amino]-1 5-H pyrido[3',4':4,5]pyrrolo[3,2-c]pyridine, trichlorhydrate - dérivé N° 9.

Ce composé est préparé selon le même mode opératoire que l'exemple N° 4 à partir de chloro-1 diméthyl-4,5 5-H pyrido[3,4:4,5]pyrrolo[3,2-c]pyridine composé de formule VI dans lequel $R_1$ = $CH_3$) et la diméthylamino-3 propylamine par chauffage à 170°C pendant 6 heures en autoclave. La base obtenue après chromatographie est transformée en trichlorhydrate par action d'une solution éthanolique d'acide chlorhydrique.

Rendement : 61 % ; F > 260°C.

Calc. pour $C_{17}H_{23}N_5$ ; 3 HCl ; 1,25 $H_2O$ ;
C,47,56 ; H, 6,70 ; N, 16,31; Cl, 24,77.

Trouvé : C,47,59 ; H, 6,50 ; N, 16,45; Cl, 24,82.

RMN $H_1$ ($D_{2}O$) ; δ : 2,35 - 2,59 (m, 2H, $CH_2$-β) ; 2,87 (d, 3H, $CH_3$-4 J $CH_3$-4-$H_3$ = 1 Hz) ; 3,07 (s, 2 x 3H, N $(CH_3)_2$) ; 3,46 - 3,66 (m, 2H, $CH_2$-γ) ; 3,94 (t, 2H, $CH_2$-α) ; 4,44 (s, 3H, $CH_3$-5) ; 8,0 (d, 1H, H-3) ; 8,38 (d, 1H, H-6, $J_{6-7}$ = 7 Hz) ; 8,88 (d, 1H, $H_7$) ; 10,0 (s, 1H, H-9).

Exemple 13.

Préparation de [(diéthylamino-4 butyl-1)amino]-1 diméthyl-4,5 5-H pyrido[3',4':4,5]pyrrolo[3,2-c]pyridine, trichlorhydrate - dérivé N° 10.

Ce composé est préparé selon le même mode opératoire que l'exemple N° 4 à partir de chloro-1 diméthyl-4,5 5-H pyrido[3',4':4,5]pyrrolo[3,2-c]pyridine (composé de formule VI dans lequel $R_1$ = $-CH_3$) et de diéthylamino-4 butylamine portée au reflux pendant 6 heures. Après chromatographie, la base est transformée en trichlorhydrate par

action d'une solution éthanolique d'acide chlorhydrique.

Rendement : 62,5 % ; F = 250°C.

Calc. pour $C_{20}H_{29}N_5$ ; 3 HCl ; 1,5 $H_2O$ :

C, 50,47; H, 7,41 ; N, 14,71 ; Cl, 22,35.

Trouvé : C, 50,58; H, 7,18 ; N, 14,79 ; Cl, 22,33.

RMN $H_1$ ($D_{2}O$) ; δ : 1,40 (t, 2 x 3H, $CH_2$-$CH_3$) ; 1,81 - 2,12 (m, 2 x 2H, $CH_2$-β et $CH_2$-γ) ; 2,84 (d, 3H, $CH_3$-4, J $CH_3$-4-$H_3$ = 1 Hz) ; 3,23 - 3,53 (m, 3 x 2H, $CH_2$-$CH_3$ + $CH_2$-δ) ; 3,72 - 3,94 (m, 2H, $CH_2$-α) ; 4,39 (s, 3H, $CH_3$-5) ; 7,89 (d, 1H, H-3) ; 8,33 (d, 1H, H-6, J $_{6-7}$ = 9 Hz) ; 8,84 (d, 1H, H-7) ; 9,86 (s, 1H, H-9).

Exemple 14.

Préparation de [(diéthylamino-4 butyl-1)amino]-1 méthyl-4 5-H pyrido[3',4':4,5]pyrrolo[3,2-c]pyridine, trichlorhydrate - dérivé N° 11.

Ce composé est préparé selon le même mode opératoire que l'exemple N° 4 à partir de chloro-1 méthyl-4 5-H pyrido[3',4':4,5]pyrrolo[3,2-c]pyridine (composé de formule VI dans lequel $R_1$ = H) et de diéthylamino-4 butylamine portée au reflux pendant 6 heures. Après chromatographie, la base est transformée en trichlorhydrate par action d'une solution éthanolique d'acide chlorhydrique.

Rendement : 51 % ; F > 260°C.

Calc. pour $C_{19}H_{27}N_5$ ; 3 HCl ; 0,5 $H_2O$ :

C, 51,41 ; H, 7,04 ; N, 15,78 ; Cl, 23,96.

Trouvé : C, 51,35 ; H, 7,07 ; N, 15,95 ; Cl, 23,68.

RMN $H_1$ ($D_{2}O$) ; δ : 1,50 (t, 2 x 3H, $CH_2$-$CH_3$) ; 1,97 - 2,25 (m, 2 x 2H, $CH_2$ - β + $CH_2$ - γ) ; 2,53 (s, 3H, $CH_3$-4) ; 3,31 - 3,62 (m, 3 x 2H, $CH_2$-$CH_3$ + $CH_2$ - δ) ; 3,68 - 3,97 (m, 2H, $CH_2$-α) ; 7,94 (s, 1H, H-3) ; 8,20 (d, 1H, H-6, J $_{6-7}$ = 8 Hz) ; 8,88 (d, 1H, H-7) ; 9,95 (s, 1H, H-9).

Exemple 15.

Préparation de [(dipropylamino-3 propyl-1)amino]-1 méthyl-4 5-H pyrido[3',4':4,5]pyrrolo[3,2-c]pyridine, trichlorhydrate - dérivé N° 12.

Ce composé est préparé selon le même mode opératoire que l'exemple N° 4 à partir de chloro-1 méthyl-4 5-H pyrido[3',4':4,5]pyrrolo[3,2-c]pyridine (composé de formule VI dans lequel $R_1$ = H) et de dipropylamino-3 propylamine portée au reflux pendant 48 heures. Après chromatographie, la base est transformée en trichlorhydrate par action d'une solution éthanolique d'acide chlorhydrique.

Rendement : 25 % ; F : 240°C.

Calc. pour $C_{20}H_{29}N_5$ ; 3 HCl ; 0,75 $H_2O$ :

C, 51,94 ; H, 7,30 ; N, 15,14.

Trouvé : C, 52,02 ; H, 7,61 ; N, 14,93.

RMN $H_1$ ($D_2O$) ; δ : 1,05 (t, 3H, $(CH_2)_2$-$\overline{CH_3}$) ; 1,66 - 2,06 (m, 3 x 2H, $CH_2$-$CH_2$ - $CH_3$ + $CH_2$-β) ; 2,$\overline{64}$ (d, 3H, $CH_3$-4, J $CH_3$-4-H-3 = 1 $\overline{Hz)}$ ; 3,23 - 4,14 (m, 4 x 2H ; N-$\underline{CH_2}$-$CH_2$-$CH_3$ + $CH_2$-γ + $CH_2$-α) ; 8,09 (d, 1H, H-3) ; 8,28 ($\overline{d,}$ 1H, H-6, J $_{6-7}$ = 1 Hz) ; 8,83 (d, 1H, H-7) ; 10,00 (s, 1H, H-9).

Exemple 16.

Préparation de diméthyl-4,5[(dipropylamino-3 propyl-1)amino]-1 5-H pyrido[3',4':4,5]pyrrolo[3,2-c]pyridine, trichlorhydrate - dérivé N° 13.

Ce composé est préparé selon le même mode opératoire que l'exemple N° 4 à partir de diméthyl-4,5 chloro-1 5-H pyrido[3',4':4,5]pyrrolo[3,2-c]pyridine (composé de formule VI dans lequel $R_1$ = $-CH_3$) et de dipropylamino-3 propylamine portée au reflux pendant 48 heures. Après chromatographie, la base est transformée en trichlorhydrate par action d'une solution éthanolique d'acide chlorhydrique.

Rendement : 30 % ; F : 220°C.

Calc. pour $C_{21}H_{31}N_5$ ; 3 HCl ; 1,5 $H_2O$ :

C, 51,48 ; H, 7,13 ; N, 14,29.

Trouvé : C, 51,42 ; H, 7,57 ; N, 14,59.

RMN $H_1$ ($D_2O$) ; δ : 1,03 (t, 2x3H) $(CH_2)_2$-$\overline{CH_3}$) ; 1,61 - 2,00 (m, 3x2H, $CH_2$ - $\underline{CH_2}$-$CH_3$ + $CH_2$-β) ; 2,83 ($\overline{d,}$ 3H, $CH_3$-4,

J $CH_3$-4-$H_3$ = 1 Hz) ; 3,22 - 4,15 (m, 4 x 2H, N-$CH_2$-$CH_2$-$CH_3$+ $CH_2$-γ + $CH_2$-α) ; 4,38 (s, 3H, $CH_3$-5) ; 8,06 (d, 1H, H-3) ; 8,28 (d, 1H, H-6, J $_{6-7}$ = 7 Hz) ; 8,84 (d, 1H, H-7) ; 9,89 (s, 1H, H-9).

Les résultats des études pharmacologiques et toxicologiques rapportés ci-après ont mis en évidence les intéressantes propriétés des dérivés de l'invention, tant sur le plan de la toxicité et de la tolérance que sur le plan de leur activité antitumorale.

L'invention a donc encore pour objet un médicament présentant, en particulier, une activité antitumorale, caractérisé en ce qu'il contient, à titre de principe actif, un dérivé de la formule I ou de l'une de ses formes tautomères lorsqu'elles existent ainsi que l'un de ses sels d'addition avec les acides minéraux ou organiques pharmaceutiquement acceptables.

ETUDE TOXICOLOGIQUE

Les composés de l'invention bénéficient d'une bonne tolérance et d'une faible toxicité.

Ainsi, l'administration à des souris hybrides $CDF_1$ saines, en une injection unique par la voie intrapéritonéale, de doses croissantes des composés à tester (5 mg, 10 mg, 20 mg, 40 mg/kg) a permis de constater que, à ces doses, les produits étaient parfaitement tolérés puisque le taux de survivants était de 100 p. 100.

Dans les mêmes conditions, administrés à des souris $BDF_1$ saines, les taux de survivants étaient de 100 p. 100 jusqu'à 100 mg/kg pour les dérivés 1, 3 et 9 et jusqu'à 200 mg/kg pour les dérivés 2 et 8.

La toxicité a été aussi évaluée en suivant, tout au long des essais, la mortalité et l'évolution pondérale des animaux d'expérience. On a constaté, ainsi que cela est rapporté plus loin, qu'aux doses efficaces, les composés de l'invention étaient bien tolérés et que l'indice thérapeutique était nettement favorable.

ETUDE PHARMACOLOGIQUE

Elle a été réalisée in vitro et in vivo.

1) In vitro : (PAOLETTI et al., Chem. Biol. Interaction, 1979, 25, 45-58).

L'activité cytotoxique des composés de l'invention a été évaluée par des essàis in vitro. Ces essais consistent à ajouter des concentrations croissantes du composé à tester à une culture cellulaire de la lignée tumorale leucémie L 1210 en phase exponentielle de croissance. Les cellules sont incubées à 37°C dans une étuve à $CO_2$ et comptées toutes les 24 heures. Le calcul de l'ID 50 (concentration du produit exprimée en micromole $l^{-1}$ qui inhibe la prolifération cellulaire à 50 p. 100) est effectué après 48 heures de contact. Ainsi déterminées, les ID 50 des composés de l'invention sont rassemblées dans le tableau I ci-après :

TABLEAU I

| Dérivé N° | ID 50 (µM $l^{-1}$) |
|---|---|
| 1 | 0,34 |
| 3 bis | 0,16 |
| 4 | 0,13 |
| 5 | 1,4 |
| 8 | 3,8 |
| 9 | 0,30 |
| 10 | 2,0 |
| 12 | 8,5 |
| 13 | 3,2 |

2) In vivo : (GERAN et al., Cancer Chemother. 1972, 2, 07-57).

L'activité antitumorale a été testée in vivo sur différentes tumeurs selon le protocole général suivant :

Toutes les souris mâles ou femelles sont inoculées par voie intrapéritonéale au jour Jo avec une quantité déterminée de cellules tumorales viables, variable selon la tumeur.

Elles sont ensuite réparties en différents lots. Chaque lot correspondant aux animaux traités par le produit à tester à une dose déterminée est composé de 10 souris. Dans ce cas, le produit à tester est dissous dans l'eau distillée et il est administré, pendant n jours ($J_1$-$J_n$) variables selon le protocole choisi, par voie intrapéritonéale à raison de 0,1 ml par 10 g de poids des souris traitées, les doses du produit administré étant précisées dans les tableaux ci-dessous.

Le lot "Contrôle négatif", correspondant aux animaux qui ne sont pas traités par un produit, comprend 2 $\sqrt{N}$ animaux, N étant égal au nombre total de souris traitées par un produit.

Enfin, tous les essais sont conduits parallèlement à l'étude d'un lot appelé "Contrôle positif" de 10 animaux qui est traité par une molécule active, variable selon le protocole choisi, dans les mêmes conditions que les lots traités par le produit à tester, afin de valider l'essai.

L'activité antitumorale est évaluée en considérant l'augmentation du temps de survie des animaux traités par rapport à celui des animaux du lot "Contrôle négatif" selon la formule :

$$T/C = \frac{\text{Jour médian de survie des animaux traités à une dose donnée de produit}}{\text{Jour médian de survie des animaux "Contrôle négatif"}} \times 100$$

Un composé est considéré comme étant actif lorsque la valeur du rapport T/C est égale ou supérieure à 125 % selon le protocole retenu. Il est toxique lorsque cette

valeur est égale ou inférieure à 85 %. Le produit est également considéré comme toxique lorsque la variation pondérale, exprimée en grammes et calculée par la formule : Poids moyen du lot mesuré au jour 5 - poids moyen du même lot au jour 1, est égale ou inférieure à -4g .

a) Test de la leucémie P 388 (GERAN et al., Cancer Chemother, 1972, 2, 07-57).

Les essais sont effectués avec des souris hybrides $CDF_1$ inoculées par voie intrapéritonéale avec $10^6$ cellules leucémiques de la lignée P 388. Le composé à tester est administré pendant 5 jours (J1-J5). Le contrôle positif du test est le 5-fluorouracile qui est administré dans les mêmes conditions.

Le nombre d'animaux survivants est évalué au jour J 40. Un composé est considéré comme étant actif lorsque le rapport T/C est supérieur à 127 %.

| Composé | Doses mg/kg | Variation pondérale en grammes | Jour médian de survie | T/C % | Survi-vants |
|---|---|---|---|---|---|
| Dérivé N° 1 | 5 | + 0,1 | 14,1 | 132 | 0/10 |
| | 10 | + 1,5 | 14,6 | 136 | 0/10 |
| | 20 | + 1,7 | 15,9 | 148 | 0/10 |
| | 40 | 0,0 | 16,6 | 155 | 0/10 |
| | 50 | - 3,9 | 3,1 | 26 | 0/10 |
| Fluorouracile | 20 | - 0,2 | 18,8 | 175 | 0/10 |
| Contrôle | 0 | + 0,3 | 10,7 | | 0/10 |

| Composé | Doses mg/kg | Variation pondérale en grammes | Jour médian de survie | T/C % | Survivants |
|---|---|---|---|---|---|
| Dérivé N° 3 (trichlorhydrate) | 0,625 | + 1,7 | 13,1 | 119 | 0 |
| | 1,25 | + 1,7 | 14,1 | 128 | 0 |
| | 2,5 | + 2,5 | 15,0 | 136 | 0 |
| | 5,0 | + 1,5 | 16,25 | 147 | 0 |
| | 10,0 | + 0,8 | 17,75 | 161 | 0 |
| | 20,0 | - 0,9 | 20,0 | 181 | 0 |
| | 40,0 | - 0,8 | 19,3 | 175 | 1 |
| | 80,0 | - 3,7 | 7,3 | 66 | 0 |
| Fluorouracile | 20 | + 0,5 | 18,9 | 171 | 0 |
| Contrôle | - | + 1,7 | 11,05 | - | 0 |

| Composé | Doses mg/kg | Variation pondérale en grammes | Jour médian de survie | T/C % | Survi-vants |
|---|---|---|---|---|---|
| Dérivé N° 3 bis (trimaléate) | 5 | - 0,4 | 15,83 | 145 | 0/10 |
| | 10 | - 0,9 | 17,13 | 156 | 0/10 |
| | 20 | - 1,7 | 20,33 | 186 | 0/10 |
| | 40 | - 3,5 | 20,25 | 184 | 0/10 |
| | 50 | - 3,3 | 22 | 181 | 1/10 à J 42 |
| | 80 | - 3,3 | 19,88 | 181 | 0/10 |
| Fluorouracile | 20 | - 0,7 | 18,88 | 172 | 0/10 |
| Contrôle | 0 | - 0,8 | 10,95 | | 0/10 |

| Composé | Doses mg/kg | Variation pondérale en grammes | Jour médian de survie | T/C % | Survivants |
|---|---|---|---|---|---|
| Dérivé N° 4 | 1,25 | - 1,5 | 12,6 | 115 | 0/10 |
| | 2,5 | - 0,6 | 14,25 | 130 | 0/10 |
| | 5,0 | - 0,7 | 14,25 | 130 | 0/10 |
| | 10 | - 0,5 | 16,13 | 147 | 0/10 |
| | 20 | - 2,1 | 18,0 | 164 | 0/10 |
| | 40 | - 1,4 | 18,75 | 172 | 0/10 |
| | 50 | - 2,4 | 25 | 207 | 3/10 à J 42 |
| | 80 | - 4,2 | 8,9 | 81 | 0/10 |
| Fluorouracile | 20 | - 0,7 | 18,88 | 172 | 0/10 |
| Contrôle | 0 | - 0,8 | 10,95 | | 0/36 |

| Composé | Doses mg/kg | Variation pondérale en grammes | Jour médian de survie | T/C % | Survi-vants |
|---|---|---|---|---|---|
| Dérivé N° 8 | 5 | - 1,5 | 13,4 | 116 | 0 |
| | 10 | - 1,1 | 14,62 | 127 | 0 |
| | 20 | - 1,1 | 16,75 | 145 | 0 |
| | 40 | - 1,6 | 16,0 | 138 | 0 |
| | 80 | - 3,7 | 17,25 | 149 | 0 |
| Fluorouracile | 20 | - 1,1 | 20,0 | 173 | 0 |
| Contrôle | - | - 0,3 | 11,55 | - | 0 |

| Composé | Doses mg/kg | Variation pondérale en grammes | Jour médian de survie | T/C % | Survivants |
|---|---|---|---|---|---|
| Dérivé N° 9 | 5 | - 1,1 | 15,75 | 136 | 0 |
| | 10 | - 1,8 | 17,38 | 150 | 0 |
| | 20 | - 2,4 | 19,33 | 167 | 0 |
| | 40 | - 3,4 | 20,0 | 173 | 0 |
| | 80 | - 6,5 | 8,25 | 71 | 0 |
| Fluorouracile | 20 | - 1,1 | 20,0 | 173 | 0 |
| Contrôle | - | - 0,3 | 11,55 | - | 0 |

b) Test de la leucémie L 1210 (GERAN et al., Cancer Chemother, 1972, 2, 07-57).

Les essais sont effectués avec des souris hybrides $CDF_1$ inoculées avec $10^5$ cellules leucémiques de la lignée L 1210. Le composé à tester est administré pendant 5 jours ($J_1$-$J_5$). Le contrôle positif du test est le 5-fluorouracile administré dans les mêmes conditions. Le nombre d'animaux survivants est évalué au jour J 60. Un composé est considéré comme étant actif lorsque le rapport T/C est supérieur ou égal à 125 %.

| Composé | Doses mg/kg | Variation pondérale en grammes | Jour médian de survie | T/C % | Survivants |
|---|---|---|---|---|---|
| Dérivé N° 3 | 1,25 | + 0,3 | 11,75 | 128 | 0 |
| | 2,5 | + 0,3 | 12,90 | 140 | 0 |
| | 5 | + 0,5 | 14,0 | 152 | 0 |
| | 10 | + 0,5 | 16,25 | 177 | 0 |
| | 20 | - 1,7 | 15,6 | 170 | 0 |
| | 40 | - 1,1 | 9,25 | 100 | 0 |
| | 50 | - 2,4 | 8,2 | 89 | 0 |
| Fluorouracile | 20 | - 0,1 | 18 | 195 | 0 |
| Contrôle | - | + 0,6 | 9,2 | - | 0 |
| Dérivé N° 4 | 2,5 | + 0,7 | 10,9 | 118 | 0 |
| | 5 | - 0,3 | 12,75 | 138 | 0 |
| | 15 | + 0,8 | 13,75 | 149 | 0 |
| | 30 | + 0,2 | 17,25 | 188 | 0 |
| | 60 | - 2,3 | 18,0 | 196 | 1 |
| | 80 | - 2,3 | 9,1 | 99 | 0 |
| Fluorouracile | 20 | - 0,5 | 16,0 | 178 | 0 |
| Contrôle | - | + 0,6 | 9,2 | - | 0 |

c) Test du mélanome B 16 (GERAN et al., Cancer Chemother, 1972, 2, 07-57).

Les essais sont effectués avec des souris hybrides $BDF_1$ inoculées avec 0,5 ml d'un homogénat de mélanome

B 16 réalisé à partir de 1 g de tumeur dans 10 ml de sérum physiologique. Le composé à tester est administré pendant 9 jours ($J_1$-$J_9$). Le contrôle positif du test est le Cis-platine administré dans les mêmes conditions. Le nombre d'animaux survivants est évalué au jour J 60. Un composé est considéré comme étant actif lorsque le rapport T/C est supérieur ou égal à 125 %.

| Composé | Doses mg/kg | Variation pondérale en grammes | Jour médian de survie | T/C % | Survi-vants |
|---|---|---|---|---|---|
| Dérivé N° 1 | 5 | + 1,1 | 28,25 | 119 | 0 |
| | 10 | + 0,9 | 30,0 | 126 | 0 |
| | 20 | + 0,9 | 34,25 | 144 | 0 |
| Cis-platine | 0,5 | - 0,6 | 30 | 126 | 0 |
| Contrôle | - | + 0,8 | 23,8 | - | 0 |
| Dérivé N° 3 | 5 | + 0,1 | 29 | 125 | 0 |
| | 20 | - 0,6 | 29,3 | 126 | 0 |
| | 40 | - 2,2 | 9,8 | 42 | 0 |
| Cis-platine | 0,5 | - 0,9 | 31,0 | 133 | 0 |
| Contrôle | - | + 0,9 | 23,25 | - | 0 |

| Composé | Doses mg/kg | Variation pondérale en grammes | Jour médian de survie | T/C % | Survivants |
|---|---|---|---|---|---|
| Dérivé N° 4 | 20 | - 1,2 | 28 | 99 | 0 |
| | 30 | - 0,5 | 43,0 | 152 | 4 |
| | 40 | - 1,1 | 53,0 | 188 | 5 |
| | 50 | - 2,0 | 39,0 | 138 | 0 |
| | 60 | - 2,5 | 24,0 | 85 | 0 |
| Cis-platine | 0,5 | - 0,9 | 40,0 | 141 | 0 |
| Contrôle | - | + 0,3 | 28,2 | - | 0 |

d) Test du réticulosarcome M5-M5076.

Les essais sont effectués avec des souris hybrides $BDF_1$ inoculées avec $10^6$ cellules de la lignée M5-M5076. Le composé à tester est administré 5 fois successivement de façon séquentielle (J1, 5, 9, 13, 17). Le contrôle positif du test est le cyclophosphamide administré dans les mêmes conditions. Le nombre d'animaux survivants est évalué au jour J 75. Un composé est considéré comme actif lorsque le rapport T/C est supérieur ou égal à 125 %.

| Composé | Doses mg/kg | Variation pondérale en grammes | Jour médian de survie | T/C % | Survi-vants |
|---|---|---|---|---|---|
| Dérivé N° 3 | 25 | - 0,1 | 51,0 | 195 | 1 |
| | 50 | - 0,1 | 45,0 | 172 | 0 |
| | 75 | - 0,1 | 41,7 | 159 | 0 |
| | 100 | - 1,7 | 44,7 | 171 | 0 |
| | 150 | - 3,75 | 10,0 | 38 | 0 |
| Cyclophosphamide | 160 | - 4,6 | > 71 | >246 | 6 |
| Contrôle | - | + 2,1 | 26,2 | - | 0 |
| Dérivé N° 4 | 10 | + 1,9 | 28,3 | 108 | 0 |
| | 25 | + 0,9 | 41,75 | 159 | 1 |
| | 50 | - 1 | 53,0 | 202 | 4 |
| | 100 | - 3,1 | 48,0 | 182 | 2 |
| | 150 | - 5,4 | 14,0 | 53 | 0 |
| Cyclophosphamide | 160 | - 1,9 | > 60 | >229 | 9 |
| Contrôle | - | + 2,1 | 26,2 | - | 0 |

Les études toxicologique et pharmacologique qui viennent d'être rapportées ont mis en évidence les intéressantes propriétés antitumorales des composés de l'invention qui les rendent très utiles en thérapeutique.

Le médicament de l'invention peut être présenté pour l'administration orale sous forme de comprimés, comprimés dragéifiés, capsules, gouttes ou sirop. Il peut aussi être présenté pour l'administration rectale sous

forme de suppositoires et pour l'administration parentérale sous forme de soluté injectable par la voie intramusculaire ou intraveineuse.

Chaque dose unitaire contient avantageusement de 0,010 g à 0,500 g associés à des excipients et véhicules convenables, les doses administrables journellement pouvant varier de 0,020 g à 3,00 g de principe actif, en fonction du poids du malade, de son âge et de la sévérité de son état.

On donnera ci-après à titre d'exemple non limitatif quelques formulations pharmaceutiques du médicament de l'invention.

1) Comprimés dragéifiés.

| | |
|---|---|
| Dérivé N° 3 | 0,100 g |
| Excipient : | gomme laque, stéarate de magnésium, talc, gomme arabique, saccharose, cire de carnauba, indigotine. |

2) Comprimés.

| | |
|---|---|
| Dérivé N° 1 | 0,250 g |
| Excipient : | amidon, fécule, talc, carbonate de calcium, stéarate de magnésium, érythrosine. |

3) Capsules.

| | |
|---|---|
| Dérivé N° 4 | 0,125 g |
| Excipient : | talc, stéarate de magnésium, oxyde de titane. |

4) Soluté injectable.

| | |
|---|---|
| Dérivé N° 9 | 0,200 g |
| Excipient : | solvant isotonique qsp 3 ml. |

5) Suppositoires.

| | |
|---|---|
| Dérivé N° 8 | 0,150 g |
| Excipient : | triglycérides semi-synthétiques qsp 1 suppositoire. |

Pour ses propriétés antitumorales, le médicament de l'invention est indiqué dans le traitement des tumeurs solides et de leurs métastases ainsi que dans le traitement des tumeurs ascitiques.

REVENDICATIONS

1. Composés de formule :

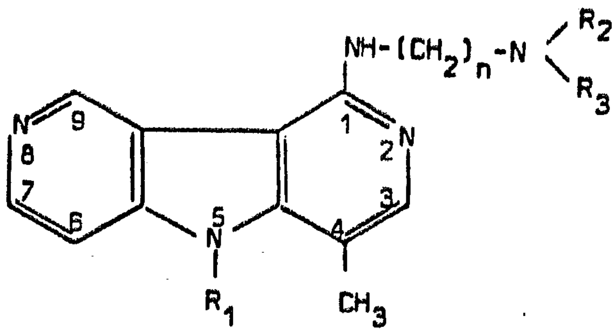

dans laquelle n représente un nombre entier de 2 à 4, $R_1$ représente l'hydrogène ou un groupe alcoyle inférieur, $R_2$ et $R_3$ sont chacun, indépendamment l'un de l'autre, l'hydrogène, un groupe alcoyle inférieur ou hydroxyalcoyle, et leurs sels d'addition avec les acides minéraux ou organiques pharmaceutiquement acceptables, ainsi que les formes tautomères lorsqu'elles existent.

2. Procédé de préparation des composés selon la revendication 1, caractérisé en ce que :

a) par réaction de l'hydrazine hydratée sur l'hydroxy-4 méthyl-5 1-H pyridone-2, on prépare l'hydrazino-4 pyridone-2 de formule (II) :

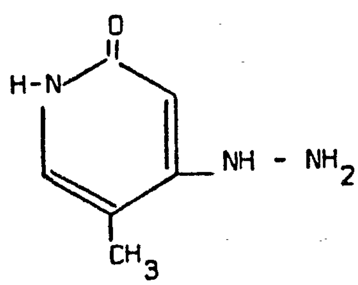

(II)

b) on fait réagir l'hydrazine de formule (II) sur la N-acétyl pipéridone-4 pour obtenir l'hydrazone correspondante de formule (III) :

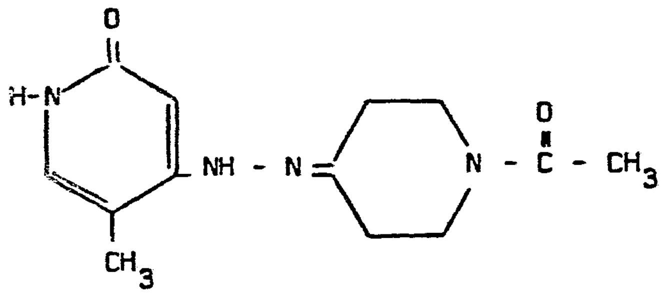

(III)

c) par la réaction de Fisher, on cyclise l'hydrazone de formule (III) pour former la tétrahydro-6,7,8,9 acétyl-8 méthyl-4 2-H, 5-H pyrido[3',4':4,5]pyrrolo[3,2-c]pyridone-1 de formule (IV) :

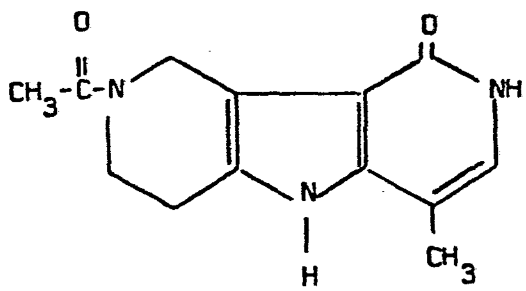

(IV)

d) on aromatise le composé de formule (IV) pour obtenir la méthyl-4 2-H, 5-H pyrido[3',4':4,5]pyrrolo[3,2-c]pyridone-1 de formule (V) :

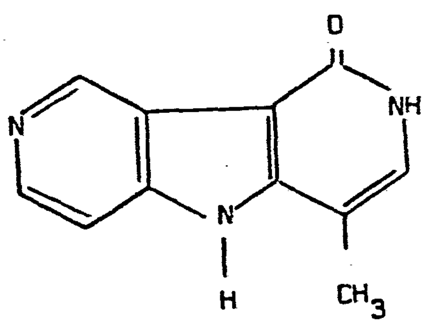

(V)

e) on soumet le composé de formule (V) à une chloration et ensuite, si nécessaire, à une réaction d'alcoylation pour obtenir un dérivé méthyl-4 5-H pyrido[3',4':4,5]pyrrolo[3,2-c]pyridine de formule (VI) :

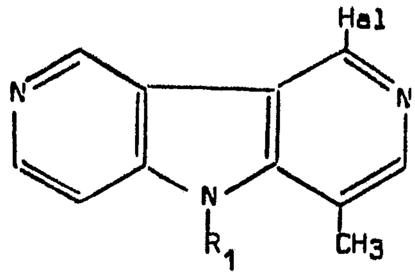

(VI)

dans laquelle $R_1$ est l'hydrogène ou un groupe alcoyle inférieur ; et

f) on condense sur le composé de formule (VI) l'amine de formule $NH_2 - (CH_2)_n - N \langle {R_2 \atop R_3}$ (n, $R_2$ et $R_3$ tels que définis ci-dessus) pour obtenir le composé de formule (I).

3. [(Diéthylamino-3 propyl-1)amino]-1 méthyl-4 5-H pyrido[3',4':4,5]pyrrolo[3,2-c]pyridine (dérivé N° 1) et ses sels pharmaceutiquement acceptables.

4. [(Diméthylamino-3 propyl-1)amino]-1 méthyl-4 5-H pyrido[3',4':4,5]pyrrolo[3,2-c]pyridine (dérivé N° 3) et ses sels pharmaceutiquement acceptables.

5. [(Diéthylamino-3 propyl-1)amino]-1 diméthyl-4,5 5-H pyrido[3',4':4,5]pyrrolo[3,2-c]pyridine (dérivé N° 4) et ses sels pharmaceutiquement acceptables.

6. Diméthyl-4,5[(éthylamino-3 propyl-1)amino]-1 5-H pyrido[3',4':4,5]pyrrolo[3,2-c]pyridine (dérivé N° 8) et ses sels pharmaceutiquement acceptables.

7. Diméthyl-4,5[(diméthylamino-3 propyl-1)amino]-1 5-H pyrido[3',4':4,5]pyrrolo[3,2-c]pyridine (dérivé N° 9) et ses sels pharmaceutiquement acceptables.

8. [(Dipropylamino-3 propyl-1)amino]-1 méthyl-4 5-H pyrido[3',4':4,5]pyrrolo[3,2-c]pyridine (dérivé N° 12) et ses sels pharmaceutiquement acceptables.

9. Diméthyl-4,5[(dipropylamino-3 propyl-1)amino]-1 5-H pyrido[3',4':4,5]pyrrolo[3,2-c]pyridine (dérivé N° 13) et ses sels pharmaceutiquement acceptables.

10. Médicament présentant en particulier des activités antitumorales, caractérisé en ce qu'il contient, à titre de principe actif un dérivé de formule (I) suivant la revendication 1 ou un sel d'addition avec un acide minéral ou organique pharmaceutiquement acceptable de celui-ci ainsi que les formes tautomères lorsqu'elles existent.

11. Médicament selon la revendication 10, caractérisé en ce qu'il contient à titre de principe actif la [(diéthylamino-3 propyl-1)amino]-1 méthyl-4 5-Hpyrido-[3',4':4,5]pyrrolo[3,2-c]pyridine (dérivé N° 1) et ses sels pharmaceutiquement acceptables.

12. Médicament selon la revendication 10, caractérisé en ce qu'il contient à titre de principe actif la [(diméthylamino-3 propyl-1)amino]-1 méthyl-4 5-H pyrido-[3',4':4,5]pyrrolo[3,2-c]pyridine (dérivé N° 3) et ses sels pharmaceutiquement acceptables.

13. Médicament selon la revendication 10, caractérisé en ce qu'il contient à titre de principe actif la [(diéthylamino-3 propyl-1)amino]-1 diméthyl-4,5 pyrido-[3',4':4,5]pyrrolo[3,2-c]pyridine (dérivé N° 4) et ses sels pharmaceutiquement acceptables.

14. Médicament selon la revendication 10, caractérisé en ce qu'il contient à titre de principe actif la (diméthyl-4,5[(éthylamino-3 propyl-1)amino]-1 5-H pyrido-[3',4':4,5]pyrrolo[3,2-c]pyridine (dérivé N° 8) et ses sels pharmaceutiquement acceptables.

15. Médicament selon la revendication 10, caractérisé en ce qu'il contient à titre de principe actif la (diméthyl-4,5[(diméthylamino-3 propyl-1)amino]-1 5-H pyrido-[3',4':4,5]pyrrolo[3,2-c]pyridine (dérivé N° 9) et ses sels pharmaceutiquement acceptables.

16. Médicament selon l'une des revendications 10 à 15, caractérisé en ce qu'il est présenté sous forme de doses unitaires, le principe actif étant associé à un

véhicule pharmaceutiquement approprié.

17. Médicament selon l'une des revendications 10 à 16, caractérisé en ce que chaque dose unitaire contient de 0,010 g à 0,500 g de principe actif, les doses administrables journellement pouvant varier de 0,010 g à 3,00 g de principe actif.

REVENDICATIONS (pour AT)

1 - Procédé de preparation de composés de formule

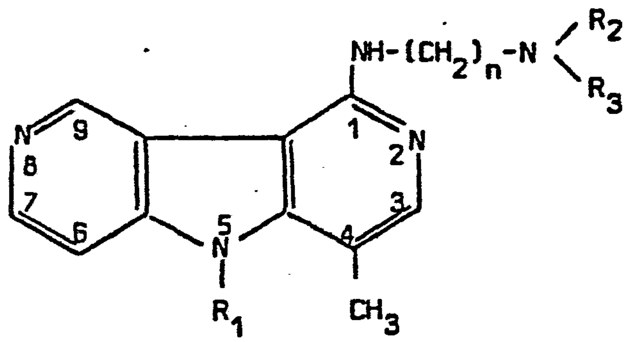

dans laquelle n représente un nombre entier de 2 à 4, $R_1$ représente l'hydrogène ou un groupe alcoyle inférieur, $R_2$ et $R_3$ sont chacun, indépendamment l'un de l'autre, l'hydrogène, un groupe alcoyle inférieur ou hydroxyalcoyle, et leurs sels d'addition avec les acides minéraux ou organiques pharmaceutiquement acceptables, ainsi que les formes tautomères lorsqu'elles existent

Procédé caractérisé en ce que

a) par réaction de l'hydrazine hydratée sur l'hydroxy-4 méthyl-5 1-H pyridone-2, on prépare l'hydrazino-4 pyridone-2 de formule (II)

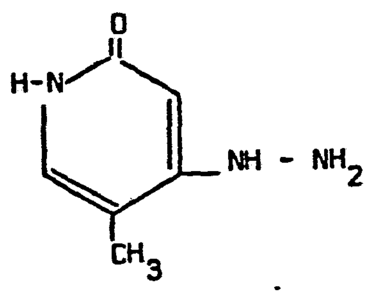

(II)

b) on fait réagir l'hydrazine de formule (II) sur la N-acétyl pipéridone-4 pour obtenir l'hydrazone correspondante de formule (III)

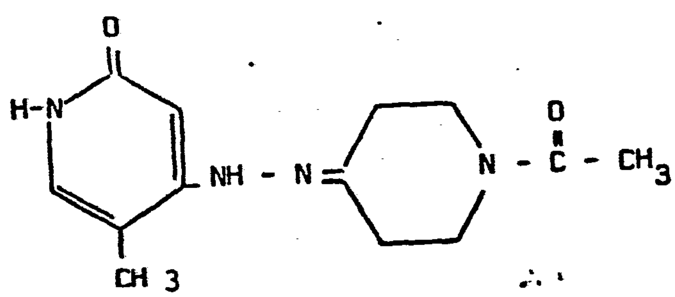

(III)

c) par la réaction de Fisher, on cyclise l'hydrazone de formule (III) pour former la tetrahydro-6,7,8,9 acétyl-8 méthyl-4 2-H, 5-H pyrido ⁅ 3', 4' : 4,5 ⁆ pyrrolo ⁅ 3,2-c ⁆ pyridone-1 de formule (IV)

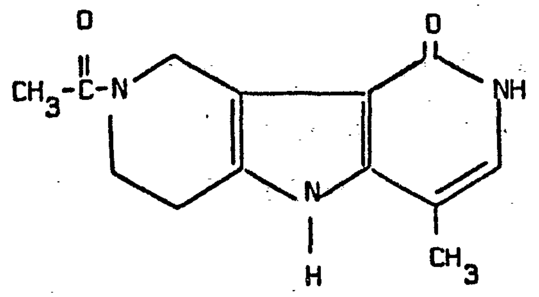

(IV)

d)on aromatise le composé de formule (IV) pour obtenir la méthyl-4 2-H, 5-H pyrido ⁅ 3', 4' : 4,5 ⁆ pyrrolo ⁅ 3,2-c ⁆ pyridone-1 de formule (V)

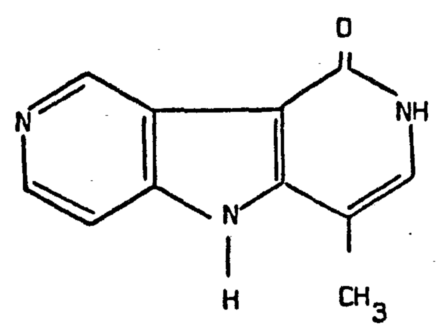

(V)

e) on soumet le composé de formule (V) à une chloration et ensuite, si nécessaire, à une réaction d'alcoylation pour obtenir un dérivé méthyl-4 5-H pyrido ⁅ 3', 4' : 4,5 ⁆ pyrrolo ⁅ 3,2-c ⁆ pyridine de formule (VI)

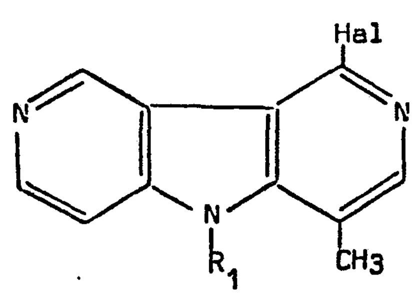

(VI)

dans laquelle $R_1$ est l'hydrogène ou un groupe alcoyle inférieur; et

f) on condense sur le composé de formule (VI) l'amine de formule $NH_2-(CH_2)n-N\begin{matrix}R_2\\R_3\end{matrix}$ (n, $R_2$ et $R_3$ tels que définis ci-dessus) pour obtenir le composé de formule (I) -

2 - Procédé de préparation d'un médicament présentant en particulier des activités antitumorales, caractérisé en ce que l'on met sous forme pharmaceutiquement acceptable un dérivé de formule (I) obtenu selon la revendication 1.

Office européen des brevets

# RAPPORT DE RECHERCHE EUROPEENNE

0197829
Numéro de la demande

EP 86 40 0604

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| A | FR-A- 913 931 (RHONE-POULENC) * Page 1, lignes 1-14 * | 1,10 | C 07 D 471/14<br>A 61 K 31/435//<br>(C 07 D 471/14<br>C 07 D 221:00<br>C 07 D 221:00<br>C 07 D 209:00 ) |
| A | FR-A-2 422 662 (ANVAR) * Revendication 1; page 20, lignes 14-35; page 21, ligne 2 * | 1,10 | |
| | | | DOMAINES TECHNIQUES RECHERCHES (Int. Cl.4) |
| | | | C 07 D 471/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 26-06-1986 | ALFARO I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82